# EUROPEAN PATENT SPECIFICATION

(11) **EP 4 461 277 B1**
(45) Date of publication and mention of the grant of the patent: **10.12.2025**
(21) Application number: 24174025.7
(22) Date of filing: 03.05.2024
(51) Int. Cl.: A61F 13/15, B65H 39/06

(54) **MACHINE AND METHOD FOR MAKING ABSORBENT SANITARY ARTICLES**
MASCHINE UND VERFAHREN ZUR HERSTELLUNG ABSORBIERENDER HYGIENEARTIKEL
MACHINE ET PROCÉDÉ DE FABRICATION D'ARTICLES SANITAIRES ABSORBANTS

(30) Priority: 08.05.2023 IT 202300009093
(43) Date of publication of application: 13.11.2024
(73) Proprietor: GDM S.p.A., 40133 Bologna (IT)
(72) Inventor: PIANTONI, Matteo, BOLOGNA (IT); SACCOMANI, Alessandro, BOLOGNA (IT); LISSANI, Gino, BOLOGNA (IT); ZAVALLONI, Alessandro, BOLOGNA (IT); FUSARPOLI, Aldo, BOLOGNA (IT)
(74) Representative: Puggioli, Tommaso

(56) References cited:
- WO-A1-2014/076626
- CN-B- 110 636 822
- JP-A- 2007 044 374
- US-A1- 2015 024 919
- US-B1- 6 730 189

## Description

This invention relates to a method and a machine for making absorbent sanitary articles such as disposable nappies for adults.

Generally speaking, in the production of absorbent sanitary articles, it is often necessary to apply discrete elements on a continuous web and such discrete elements must be suitably spaced based, for example, on the format or size of the absorbent article.

The absorbent articles concerned comprise, amongst other things, side panels or wings, on the right- and left-hand sides, for closing the nappy around the wearer's waist, which are usually obtained by cutting a continuous web or strip into segments and which are then applied on a continuous support web advancing along a machine direction.

To apply the side panels, prior art machines usually comprise a cutting unit, which receives the continuous web or strip to cut it into the aforesaid right- and left-hand segments, and transfer units located downstream of the cutting unit.

The transfer units pick up or receive the individual segments from the cutting unit, space them along both the machine direction as a function of the length of the absorbent article, and a cross direction transverse to the machine direction as a function of the width of the absorbent article, and apply them to the supporting web so that each right-hand panel has a corresponding left-hand panel.

Documents WO2008/155618 and WO2014/076626 each disclose, for example, a method and a machine for making absorbent articles where, in addition to the above, the pairs of right- and left-hand side wings are arranged beforehand on an alignment roller which applies them on the support web.

The spacing between pairs of panels on the support web depends, as mentioned above, on the size of the absorbent article being made, and the prior art machines either have a very complex way of enabling the right- and left-hand panels to be aligned or placed in phase and suitably spaced in both the machine and cross directions or they necessitate replacement of some of their components when changing over to making an absorbent article of a different size.

Other prior art solutions are described, for example, in US2015024919 and US6730189B1.

The prior art solutions are therefore cumbersome and/or complex and/or costly.

In this context, the intention is to provide a method and a machine for making absorbent sanitary articles and capable of overcoming at least some of the disadvantages of the prior art and of carrying out the aforesaid intention.

In particular, this invention has for an aim to provide a method and a machine for making absorbent sanitary articles which allow discrete elements to be applied on a continuous web and suitably spaced in a simple and inexpensive manner.

This aim is achieved by a method and a machine for making absorbent sanitary articles, comprising the technical features described in one or more of the accompanying claims. The dependent claims correspond to possible different embodiments of the invention.

According to a first aspect, this invention relates to a method for making absorbent sanitary articles, each comprising a first discrete element and a second discrete element.

For example, the absorbent articles may be adult nappies and the first and the second discrete elements may be the right- and left-hand side panels for closing the nappies round the waist.

Nappies for adults are generally characterized by side panels which are approximately half as long as the nappy itself.

The method comprises a step of feeding a succession or series of first discrete elements along a first feed path.

The method comprises a step of feeding a succession or series of second discrete elements along a second feed path.

The method comprises applying each first discrete element of the succession of first discrete elements on a support web which is movable parallel to its own longitudinal axis in a feed direction along a third feed path.

According to the method, the first discrete elements are applied on the support web at a first applicator station.

The method comprises applying each second discrete element of the succession of second discrete elements on the support web at a second applicator station located downstream of the first applicator station in the feed direction of the support web.

The support web may be a single web or a composite web, that is to say, a web comprising two or more components of what will be finished absorbent articles. Generally speaking, the composite web, with the side panels and any other components that may be applied even after the side panels, is then normally cut into separate absorbent articles.

The method comprises adjusting a length of the third feed path between the first applicator station and the second applicator station, so that the second discrete element is applied at a predefined position relative to the first discrete element.

For example, adjusting the length of the third feed path between the first applicator station and the second applicator station is carried out as a function of a predefined application position of the second discrete element relative to the first discrete element along the support web.

The length of the third feed path between the first applicator station and the second applicator station determines the length of the support web between the first applicator station and the second applicator station and can be set, for example, based on a size of the absorbent article.

Preferably, the method comprises cutting a continuous web upstream of the first and the second feed path to make the first discrete elements and the second discrete elements from the continuous web and feeding the first discrete elements to the first feed path and the second discrete elements to the second feed path. Such a configuration is preferably used to make the side panels from a single web, waste free; all the material of the continuous web or strip is used to make the side panels without producing any waste in what is known as a *zero-waste* application.

For example, the continuous web may be cut by a succession of cuts transverse to a main direction of extension of the continuous web itself to obtain side panels that will be symmetrical relative to the support web when they are applied thereto.

Preferably, the method comprises translating the first discrete elements along the first feed path in a first direction transverse to the first feed path and/or translating the second discrete elements along the second feed path in a second direction transverse to the second feed path. Advantageously, that way, the discrete elements can be positioned, in the cross direction, at a position suitable for them to be applied to the support web.

The method may comprise accelerating the first discrete elements along the first feed path and/or accelerating the second discrete elements along the second feed path to bring them to an application speed suitable for them to be applied to the support web moving along the respective feed path.

Preferably, the method comprises holding the first discrete elements on the support web by suction at least between the first applicator station and the second applicator station.

Preferably, the first and the second discrete element are applied on the support web in such a way that each second discrete element is symmetrical to a corresponding first discrete element about the longitudinal axis of the support web so they belong to the same absorbent article once the composite web, as mentioned above, is cut into separate absorbent articles.

Preferably, the method is advantageously applicable when the sum of the length of the first discrete element, measured along the support web, and the length of the second discrete element, measured along the support web, is different from the length of the absorbent sanitary article, measured along the support web. In particular, the method is advantageous for applying the side panels of the absorbent article when the total length of the right-hand panel and of the left-hand panel is different from the length of the absorbent article.

According to an aspect, this invention relates to a machine for making absorbent sanitary articles.

Preferably, the machine implements the method of the first aspect to which express reference will also be made without losing in generality.

The machine comprises a first transfer unit at least partly defining a first feed path for a succession of first discrete elements movable along the first feed path. Preferably, the first transfer unit rotates about a first axis of rotation transverse to the first feed path.

The machine comprises a second transfer unit at least partly defining a second feed path for a succession of second discrete elements movable along the second feed path. Preferably, the second transfer unit rotates about a second axis of rotation transverse to the second feed path.

The machine comprises a first applicator station located downstream of the first transfer unit along the first feed path for applying each first discrete element of the succession of first discrete elements on a support web movable in a feed direction along the third feed path.

The machine comprises a second applicator station, located downstream of the first applicator station along the feed path of the support web and downstream of the second transfer unit along the second feed path, for applying each second discrete element of the succession of second discrete elements on the support web.

The machine comprises an adjustment device for adjusting the third feed path between the first applicator station and the second applicator station.

The adjustment device at least partly defines the third feed path and is configured to adjust a length of the third feed path between the first applicator station and the second applicator station, so that the second discrete element is applied at a predefined position relative to the first discrete element.

The length of the third feed path between the first applicator station and the second applicator station determines the length of the support web between the first applicator station and the second applicator station and can be set, for example, based on a size of the absorbent article.

The adjustment device may comprise, for example, a first rotary drum at the first applicator station, a second rotary drum at the second applicator station and at least one adjustment drum, interposed between the first and the second rotary drum, for adjusting the third feed path.

The drums of the adjustment device have parallel axes of rotation transverse to the third feed path.

The adjustment drum is movable relative to the first rotary drum and to the second rotary drum between a first operating position and a second operating position corresponding respectively to a maximum length of the third feed path between the first applicator station and the second applicator station, and a minimum length of the third feed path between the first applicator station and the second applicator station.

For example, at the first operating position, the adjustment drum is away from the first and the second rotary drum, whilst at the second operating position, it is close to them.

Preferably, the first rotary drum, the second rotary drum and the adjustment drum are provided with suction means to hold the support web and to hold the first and second discrete elements in place on the support web.

The machine may comprise, between the first rotary drum and the adjustment drum, a first suction box which delimits a first stretch of the third feed path.

The machine may comprise, between the adjustment drum and the second rotary drum, a second suction box which delimits a second stretch of the third feed path.

The whole of the third feed path between the first applicator station and the second applicator station is provided with suction for holding the first discrete element and/or the second discrete element on the support web. The machine may comprise a cutting unit, located upstream of the first and the second transfer unit for cutting a continuous web upstream of the first and the second feed path to make the first discrete elements and the second discrete elements from the continuous web.

The cutting unit and the first and second transfer units may be positioned relative to each other in such a way that the cutting unit feeds the first discrete elements to the first feed path and the second discrete elements to the second feed path.

Preferably, the first transfer unit comprises at least a first working unit movable along a first direction transverse to the first feed path for moving the first discrete element. The first transfer unit is configured so that the first movable working unit moves the first discrete element in translation along the first transverse direction in a substantially known manner.

Preferably, the second transfer unit comprises at least a second working unit movable along a second direction transverse to the second feed path for moving the second discrete element. The second transfer unit is configured so that the second movable working unit moves the second discrete element in translation along the first transverse direction in a substantially known manner.

The movable working units of the first and second transfer units adjust the position of the first and second discrete elements in a direction transverse to the feed paths.

The movable working units of the first and second transfer units adjust the position of the first and second discrete elements in a direction transverse to the machine direction, that is to say, they position the first and second discrete elements in the cross direction.

Preferably, the machine comprises a first acceleration unit, located downstream of the first transfer unit and upstream of the first applicator station along the first feed path for adjusting a transfer speed of the first discrete elements before they are applied on the support web.

In practice, preferably, as they advance towards the first applicator station, the first discrete elements are formed in the cutting station, suitably positioned in the cross direction on the first transfer wheel and accelerated to the application speed by the first acceleration unit.

Preferably, the machine comprises a second acceleration unit, located downstream of the second transfer unit and upstream of the second applicator station along the second feed path for adjusting a transfer speed of the second discrete elements before they are applied on the support web.

In practice, preferably, as they advance towards the second applicator station, the second discrete elements are formed in the cutting station, suitably positioned in the cross direction on the second transfer wheel and accelerated to the application speed by the second acceleration unit.

The machine for making absorbent sanitary articles is simple and lightweight, in particular because in the specific case of applying the side panels, the right-hand panel and the left-hand panel are placed in phase by a device as simple as a path regulator.

The layout of the machine is also simple, without particularly complex wheel systems to manage acceleration and phasing, for example. Maintenance, when required, is also easy in that all the units are easy to access.

Moving the discrete elements on suction drums and/or along suction paths makes it easier for the panels to not be disarranged, in particular in the case where the adjustment path defined between the first and the second applicator station is provided with uninterrupted suction.

In a preferred example, any changes to the path of the support web can be made by moving the adjustment drum.

When changing over to a different size, the setting of the adjustment drum changes; the position of the adjustment drum is chosen, in particular, to define the placement position where the second discrete element is placed on the support web as a function of the size.

The second applicator station may be followed by an outfeed conveyor for transferring the support web provided with the first and second discrete elements to the processing stations downstream.

The method and machine according to the aforesaid aspects are particularly advantageous when the discrete elements to be applied to the support web are large relative to the product to be made. In particular, varying the length of the web between the application of the first discrete element and the application of the second discrete element avoids having to construct complex machines to position and also phase the discrete elements, that is to say, it avoids the need for otherwise very complex laws of motion.

Further features and advantages of the above aspects are more apparent in the exemplary, hence non-limiting description of a method and a machine for applying web segments to a continuous web.

The description is set out below with reference to the accompanying drawings which are provided solely for purposes of illustration without restricting the scope of the invention and in which:
- Figure 1 is a schematic front view illustrating a machine for making absorbent sanitary articles according to this invention, in a first operating configuration;
- Figure 2 illustrates the machine of Figure 1 in a schematic front view, in a second operating configuration;
- Figure 3 is a schematic front view showing a cutting pattern in a cutting unit of a machine for making absorbent sanitary articles according to this invention;
- Figure 4 schematically illustrates steps of a method for making absorbent sanitary articles according to the description;
- Figure 5 schematically illustrates steps of a method for making absorbent sanitary articles according to the description;
- Figure 6 illustrates a semifinished product obtainable with a machine for making absorbent sanitary articles according to the description.

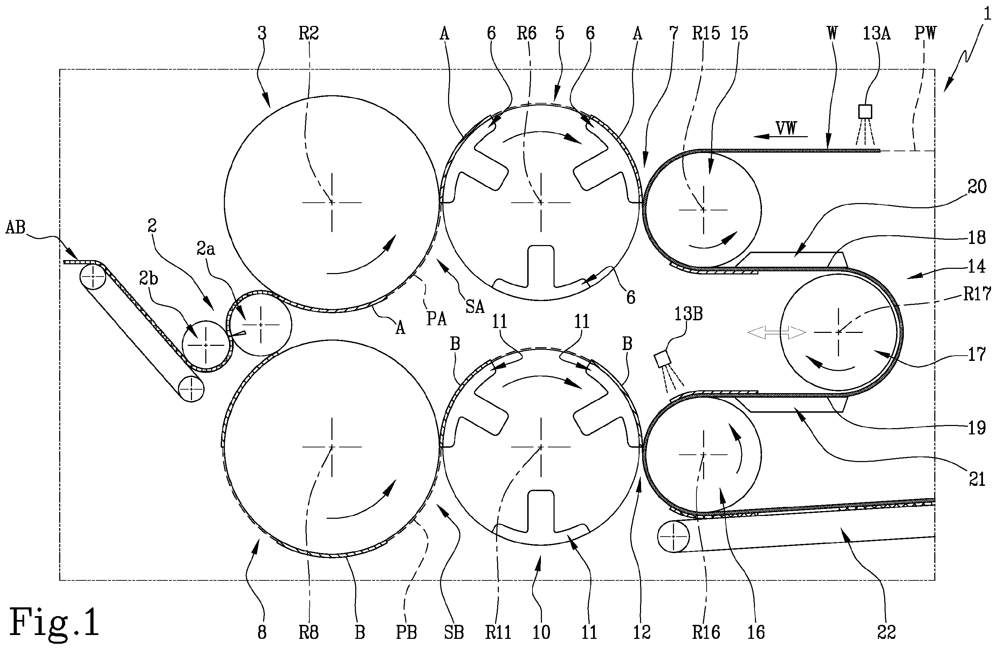

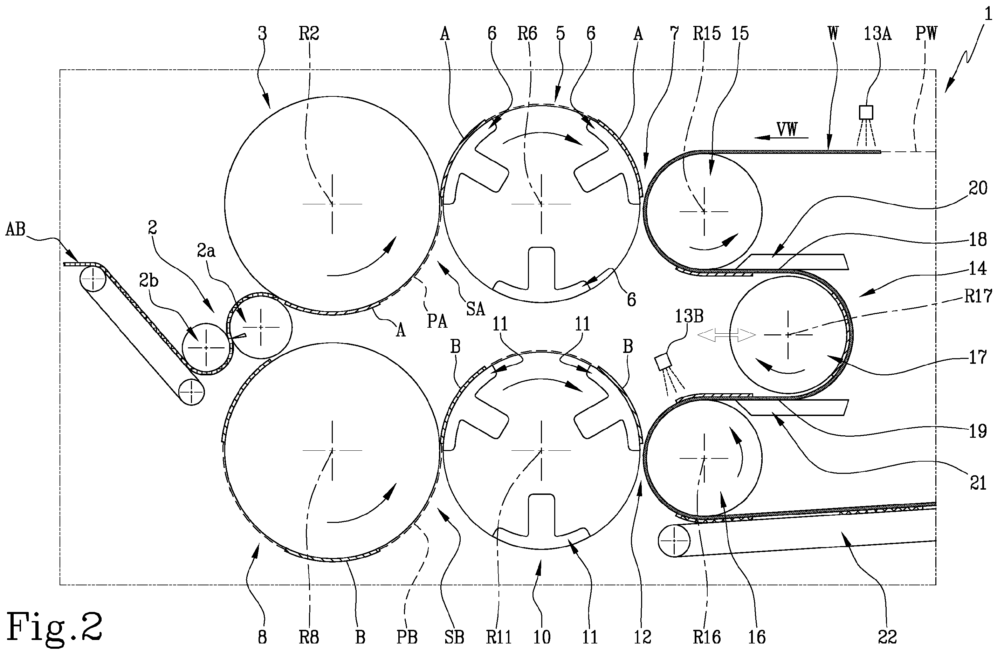

With reference to Figures 1 and 2, the numeral 1 denotes a machine for making wearable absorbent sanitary article such as, for example, baby nappies, adult nappies and the like. Generally speaking, the absorbent articles concerned are of the type comprising a first and a second discrete element A and B which are applied on a support web W. In the machine 1, the support web W is movable parallel to its own longitudinal axis in a feed direction VW along a feed path PW.

The support web W may be a composite web, that is, a web comprising a plurality of pre-assembled components and also for this reason, the web provided with the discrete elements A and B is also denoted by the same reference character W.

The web W is, in practice, a semifinished product from which the absorbent sanitary articles will be made.

The machine 1 may form part of a plant, not illustrated, for the production of absorbent sanitary articles in which the machine 1 carries out intermediate steps of a complete process for the production of absorbent sanitary articles.

The first and the second discrete element A and/or B may be, for example, side panels of the absorbent article for closing the article round the waist, web segments with desired properties, such as an *acquisition and distribution layer* (ADL), applied to the absorbent article, an impermeable web segment or a comfort enhancing web segment for the absorbent article.

In this disclosure. express reference is made, without thereby losing in generality, to an absorbent article comprising a first and a second discrete element A and B constituting, respectively, a side panel A and a side panel B which will form part of a closing system allowing the absorbent sanitary article to be closed round the waist of a wearer, not illustrated.

In particular, express reference will also be made to a machine for making adult nappies comprising the first and second side panels A and B.

Adult nappies are, for example, between 800 mm and 1000 mm long, with side panels between 400 mm and 500 mm long; that is to say, the side panels are very large in relation to the maximum length of the absorbent sanitary article. It should be considered, for example, that the side wings of a baby nappy, which is between 300 mm and 600 mm in length, are between 50 mm and 80 mm long.

With reference in particular to Figures 1 and 2, the machine comprises a feed path PA for the side panels A and a feed path PB for the side panels B.

The feed direction of the side panels A and B along the respective feed paths PA and PB, from left to right looking, in particular, at Figures 1 and 2, defines what is known as the *machine direction.*

As illustrated, for example, in Figures 1 to 3, the machine 1 comprises a cutting unit 2, located upstream of the paths PA and PB, for cutting a continuous web AB and making the discrete elements A and the discrete elements B from the continuous web AB.

The cutting unit 2, of substantially known type, is represented schematically as two counter-rotating rollers 2a, 2b, where, for example, one roller 2a comprises a cutting edge and the other, 2b, constitutes an anvil.

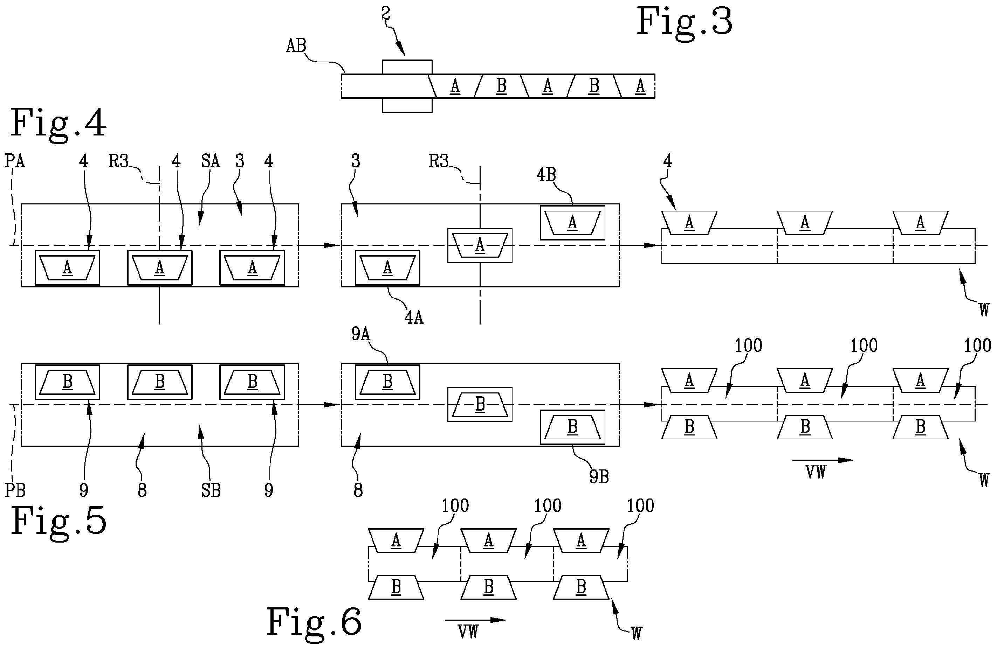

Figure 3 shows a preferred cutting pattern for making the side panels A and B. The side panels A and B are all the same and are made by cutting the web AB transversely to its feed direction. In the example illustrated, the side panels A and B are in the shape of an isosceles trapezium and the web AB is cut without producing any waste, in a manner known as *zero waste.*

The cutting unit 2 is positioned relative to the paths PA and PB in such a way that the cutting unit 2 feeds the discrete elements A to the feed path PA and the discrete elements B to the feed path PB.

The machine 1 comprises a transfer unit 3 for transferring the discrete elements A and which at least partly defines the feed path PA.

Figure 4 represents a plan view of the transfer unit 3 for descriptive purposes.

In the example illustrated, the transfer unit 3 rotates anticlockwise about an axis of rotation R3 transverse to the feed path PA.

The transfer unit 3 comprises a plurality of working units 4, movable along a direction which is transverse to the feed path PA and preferably parallel to the axis R3.

The working units 4 are movable along the axis R3 between a pickup point 4A and a release point 4B.

The working units 4 move the discrete elements A in translation in an axial direction between the pickup position 4A and the release position 4B as they advance along the path PA.

During a rotation of the transfer unit 4, the side panels A are picked up at the position 4A and released at the position 4B.

The movable working units 4 of the transfer unit 3 adjust the position of the discrete elements A in a cross direction transverse to the machine direction.

The machine 1 comprises an acceleration unit 5, located downstream of the transfer unit 3 along the feed path PA.

The acceleration unit 5 adjusts the transfer speed of the discrete elements A before they are applied on the support web W.

In the example illustrated, the acceleration unit 5 comprises three working units 6 which oscillate about an axis of rotation R6 to transfer the discrete elements A from the transfer unit 3 to an applicator station 7, where the discrete elements A are applied on the support web W.

A glue dispenser, schematically represented as a block 13A, is configured to dispense adhesive on the web W before the side panels A are applied. Preferably, the discrete elements A are held on the transfer unit 3 and on the oscillating working units 6 by suction in a substantially known manner. The applicator station 7 is located downstream of the transfer unit 3 along the feed path PA.

As they advance towards the applicator station 7, the discrete elements A are formed in the cutting station 2, suitably positioned in the cross direction on the transfer wheel 3 and accelerated to the application speed by the acceleration unit 5.

The acceleration unit 5 can also modify the spacing of the discrete elements A in the succession SA, that is to say, the distance between consecutive discrete elements A, to obtain the spacing at which the discrete elements A are applied on the support web W.

Figure 4 shows the web W provided with the side panels A, for example, glued to the web W, as positioned in the applicator station 7.

The machine 1 comprises a transfer unit 8 for transferring the discrete elements B and which at least partly defines the feed path PB.

Figure 5 represents a plan view of the transfer unit 8 for descriptive purposes.

In the example illustrated, the transfer unit 8 rotates anticlockwise about an axis of rotation R8 transverse to the feed path PB.

The transfer unit 8 comprises a plurality of working units 9, movable along a direction which is transverse to the feed path PB and preferably parallel to the axis R8.

The working units 9 are movable along the axis R8 between a pickup point 9A and a release point 9B.

The working units 9 move the discrete elements B in translation in an axial direction between the pickup position 9A and the release position 9B as they advance along the path PB.

During a rotation of the transfer unit 8, the side panels B are picked up at the position 4A and released at the position 4B.

The movable working units 9 of the transfer unit 8 adjust the position of the discrete elements A in a cross direction transverse to the machine direction.

The machine 1 comprises an acceleration unit 10, located downstream of the transfer unit 8 along the feed path PB.

The acceleration unit 10 adjusts the transfer speed of the discrete elements B before they are applied on the support web W.

In the example illustrated, the acceleration unit 10 comprises three working units 11 which oscillate about an axis of rotation R11 to transfer the discrete elements B from the transfer unit 8 to an applicator station 12, where the discrete elements B are applied on the support web W. Preferably, the discrete elements B are held on the transfer unit 8 and on the oscillating working units 11 by suction in a substantially known manner.

The applicator station 12 is located downstream of the transfer unit 8 along the feed path PB.

The applicator station 12 is located downstream of the applicator station 7 along the feed path PW of the web W; that is to say, the discrete elements B are applied on the web W after the discrete elements A.

A glue dispenser, schematically represented as a block 13B, is configured to dispense adhesive on the web W before the side panels B are applied.

As they advance towards the applicator station 12, the discrete elements B are formed in the cutting station 2, suitably positioned in the cross direction on the transfer wheel 8 and accelerated to the application speed by the acceleration unit 10.

The acceleration unit 10 can also modify the spacing of the discrete elements B in the succession B, that is to say, the distance between consecutive discrete elements B, to obtain the spacing at which the discrete elements B are applied on the support web W.

Figure 5 shows the web W provided with the side panels A, applied in the applicator station 7, and with the side panels B, for example, glued to the web W, as positioned in the applicator station 12.

The machine comprises an adjustment device 14 for adjusting the feed path PW of the support web W between the applicator station 7 and the applicator station 12.

The adjustment device 14 at least partly defines the feed path PW and is configured to adjust the length of the feed path PW between the applicator station 7 and the applicator station 12 so that the discrete element B is applied at a predefined position relative to the discrete element A.

For example, the adjustment device 14 allows adjusting the length of the feed path PW between the applicator station 7 and the applicator station 12 as a function of a predefined application position of the discrete element B relative to the discrete element A on the support web W.

The length of the feed path PW between the applicator station 7 and the applicator station 12 determines the length of the support web W between the applicator station 7 and the applicator station 12 and can be set, for example, based on a size of the absorbent article.

Adjusting the length of the feed path PW between the applicator station 7 and the applicator station 12 determines the application position of the discrete elements B so that each discrete element B is positioned symmetrically to a corresponding discrete element A.

In the example illustrated, the adjustment device 14 comprises a rotary drum 15 which is rotatable about an axis of rotation R15 in anticlockwise direction.

The rotary drum 15 is located at the applicator station 7.

The rotary drum 15 defines the applicator station 7 in cooperation with the acceleration unit 5.

The adjustment device 14 comprises a rotary drum 16 which is rotatable about an axis of rotation R16 in anticlockwise direction.

The rotary drum 16 is located at the applicator station 12.

The rotary drum 16 defines the applicator station 12 in cooperation with the acceleration unit 10.

Preferably, the axes of rotation R15 and R16 are parallel to each other and parallel to the axes of rotation R3, R6, R8 and R11.

The adjustment device 14 comprises an adjustment drum 17 interposed between the rotary drums 15 and 16 and rotatable about an axis of rotation R17.

The axis of rotation R17 is preferably parallel to the axes of rotation R15 and R16 and transverse to the path PW.

The adjustment drum 17 is movable between a first operating position, illustrated for example in Figure 1, and a second operating position, illustrated for example in Figure 2.

At the first operating position, the adjustment drum 14 determines a maximum length of the feed path PW between the applicator station 7 and the applicator station 12.

At the second operating position, the adjustment drum 14 determines a minimum length of the feed path PW between the applicator station 7 and the applicator station 12.

The adjustment drum 17 is movable between the first and the second operating position relative to the rotary drum 15 and to the rotary drum 16. The web W is partly wrapped round the adjustment drum 15, the adjustment drum 17 and the adjustment drum 16 and moving the adjustment drum 17 causes, in particular, the two branches 18, 19 of the feed path PW between the applicator station 7 and the applicator station 12 to be lengthened or shortened.

Shown represented on the right-hand side of Figure 5 and in Figure 6 are two embodiments of the web W obtained respectively with the adjustment drum 17 at a position further away from the rotary drums 15 and 16 and at a position closer to them.

The length of the branches 18 and 19 depends on the position of the adjustment drum 17.

Adjusting the position of the adjustment drum 17 allows aligning the panels A and B on the web W, upstream, as they enter the transfer units 3 and 8, out of phase, that is, behind one another, as made by the cutting unit 2.

The machine 1 uses the acceleration units 5 and 10 to feed the discrete elements A and B so that their placement speed is consistent with the speed of the support web W and uses the adjustment device 14 to phase each discrete element A with a corresponding discrete element B on the web W.

The position of the adjustment drum 17 is chosen, for example, as a function of the size of the absorbent article.

The rotary drum 15, the rotary drum 16 and the adjustment drum 17 are provided with suction means to hold the support web W and to hold the discrete elements A and B on the support web W.

In the embodiment illustrated, the machine 1 comprises, between the rotary drum 15 and the adjustment drum 17, a suction box 20 at the stretch 18 of the feed path PW.

The machine 1 comprises, between the adjustment drum 17 and the rotary drum 16, a suction box 21 at the stretch 19 of the feed path PW.

Thus, the whole of the feed path between the applicator station 7 and the applicator station 12 is provided with suction for holding the side panel A and/or the side panel B on the support web PW, preventing them from being disarranged.

The suction boxes 20, 21 are configured to allow the adjustment drum 17 to move while ensuring that the stretches 18 and 19 are provided with suction whatever the position of the adjustment drum 17.

In the embodiment illustrated, the machine 1 comprises an outfeed conveyor 22 for transferring the support web W provided with the discrete elements A and B to the processing stations downstream, not illustrated. Described below is a method for making absorbent sanitary articles, each comprising a discrete element A and a discrete element B, suitably positioned relative to each other. For convenience, the method is described with reference also to a machine like that illustrated in Figures 1 and 2, without thereby losing in generality.

With reference to Figures 4 and 5, the numeral 100 denotes distinct stretches of the composite web from which the absorbent articles will be obtained in further processing stations and steps, not illustrated.

The method comprises feeding a succession or series SA of discrete elements A along the feed path PA and feeding a succession or series SB of discrete elements B along the feed path B.

The method comprises applying each discrete element A of the succession SA on the support web W, which is movable parallel to its own longitudinal axis in the feed direction VW along the feed path PW. According to the method, the discrete elements A are applied on the support web W in the applicator station 7.

The method comprises applying each discrete element B of the succession SB of discrete elements B on the support web W in the second applicator station 12, located downstream of the applicator station 7 in the feed direction VW of the support web W.

The method comprises adjusting a length of the feed path VW between the applicator station 7 and the applicator station 12 so that the discrete element B is applied at a predefined position relative to the discrete element A.

The discrete elements A and B are applied on the support web W in such a way that each discrete element B is symmetric to a corresponding discrete element A about the longitudinal axis of the support web W.

Each discrete element or side panel A and the one symmetric to it B belong to the same absorbent article when the composite web W is cut into distinct absorbent articles.

The method comprises cutting the continuous web AB upstream of the feed paths PA, PB to make the discrete elements A, B and feeding the discrete elements A to the feed path PA and the discrete elements B to the feed path PB.

The continuous web AB is preferably cut by a succession of cuts transverse to a main direction of extension of the continuous web AB itself so as to obtain identical side panels A, B that will be positioned symmetrically along the support web W.

The method comprises moving the discrete elements A in translation along the feed path PA and/or moving the discrete elements B in translation along the feed path PB in the cross direction to place them at a position suitable for them to be applied on the support web W. The method comprises accelerating the discrete elements A along the feed path PA and/or accelerating the discrete elements B along the feed path PB in the cross direction to place them at a position suitable for them to be applied on the support web W.

The method comprises holding the discrete elements A on the support web W by suction at least between the applicator station 7 and the applicator station 12 so that they are not disarranged as the web W moves.

## Claims

1. A method for making absorbent sanitary articles (100), each comprising a first discrete element (A) and a second discrete element (B), the method comprising:
feeding a succession (SA) of first discrete elements (A) along a first feed path (PA);
feeding a succession (SB) of second discrete elements (B) along a second feed path (PB);
applying each first discrete element (A) of the succession (SA) of first discrete elements (A) on a support web (W) having a longitudinal axis and
movable in a feed direction (VW) along a third feed path (PW), the first discrete elements (A) being applied on the support web (W) at a first applicator station (7);
applying each second discrete element (B) of the succession (SB) of second discrete elements (B) on the support web (W) at a second applicator station (12) located downstream of the first applicator station (7) in the feed direction (VW) of the support web (W), the method being **characterized in that** it comprises adjusting a length of the third feed path (PW) between the first applicator station (7) and the second applicator station (12), so that each second discrete element (B) is applied on the support web (W) at a predefined position relative to a respective first discrete element (A), the length of the third feed path (PW) between the first applicator station (7) and the second applicator station (12) determining a length of the support web (W) between the first applicator station (7) and the second applicator station (12).

2. The method according to claim 1, wherein the first and the second discrete element (A, B) are applied on the support web (W) in such a way that each second discrete element (B) is symmetric to a corresponding first discrete element (A) about the longitudinal axis of the support web (W).

3. The method according to claim 1 or 2, comprising cutting a continuous web (AB) upstream of the first and the second feed path (PA, PB) to make the first discrete elements (A) and the second discrete elements (B) from the continuous web (AB) and feeding the first discrete elements (A) to the first feed path (PA) and the second discrete elements (B) to the second feed path (PB).

4. The method according to claim 3, wherein cutting the continuous web (AB) produces a succession of cuts transverse to a main direction of extension of the continuous web (AB).

5. The method according to any one of the preceding claims, comprising translating the first discrete elements (A) along the first feed path (PA) in a first direction (R2) transverse to the first feed path (PA) and/or translating the second discrete elements (B) along the second feed path (PB) in a second direction (R8) transverse to the second feed path (PB).

6. The method according to any one of the preceding claims, comprising accelerating the first discrete elements (A) along the first feed path (PA) and/or accelerating the second discrete elements (B) along the second feed path (PB).

7. The method according to any one of the preceding claims comprising holding the first discrete elements (A) on the support web (W) by suction at least between the first applicator station (7) and the second applicator station (12).

8. The method according to any one of the preceding claims, wherein the first discrete element (A) is a first side panel of the absorbent sanitary article (100) and the second discrete element (B) is a second side panel of the absorbent sanitary article (100).

9. The method according to any one of the preceding claims, wherein the sum of a length of the first discrete element (A), measured along the support web (W), plus a length of the second discrete element (B), measured along the support web (W), is different from the length of the absorbent sanitary article (100) measured long the support web (W).

10. The method according to any one of the preceding claims, wherein adjusting the length of the third feed path (PW) between the first applicator station (7) and the second applicator station (12) is carried out as a function of a predefined application position of the second discrete element (B) relative to the first discrete element (A) along the support web (W).

11. A machine for making absorbent sanitary articles (100), comprising:
a first transfer unit (3) at least partly defining a first feed path (PA) for a succession (SA) of first discrete elements (A) movable along the first feed path (PA);
a second transfer unit (8) at least partly defining a second feed path (PB) for a succession (SB) of second discrete elements (B) movable along the second feed path (PB);
a first applicator station (7) located downstream of the first transfer unit (3) along the first feed path (PA) for applying each first discrete element (A) of the succession (SA) of first discrete elements (A) on a support web (W) having a longitudinal axis and movable in a feed direction (VW) along a third feed path (PW);
a second applicator station (12) located downstream of the first applicator station (7) in the feed direction (VW) of the support web (W) and downstream of the second transfer unit (8) along the second feed path (PB) for applying each second discrete element (B) of the succession (SB) of second discrete elements (B) on the support web (W), the machine being **characterized in that** it comprises an adjustment device (14) for adjusting a length of the third feed path (PW) between the first applicator station (7) and the second applicator station (12), so that each second discrete element (B) is applied on the support web (W) at a predefined position relative to a respective first discrete element (A), the adjustment device (14) at least partly defining the third feed path (PW), the length of the third feed path (PW) between the first applicator station (7) and the second applicator station (12) determining a length of the support web (W) between the first applicator station (7) and the second applicator station (12).

12. The machine according to claim 11, comprising a cutting unit (2) located upstream of the first and the second transfer unit (3, 8) for cutting a continuous web (AB) upstream of the first and the second feed path (PA, PB) to make the first discrete elements (A) and the second discrete elements (B) from the continuous web (AB), the cutting unit (2) and the first and second transfer units (3, 8) being positioned relative to each other in such a way that the cutting unit (2) feeds the first discrete elements (A) to the first feed path (PA) and the second discrete elements (B) to the second feed path (PB).

13. The machine according to claim 11 or 12, wherein the first transfer unit (3) comprises at least a first working unit (4) movable along a first direction (R3) transverse to the first feed path (PA) for moving the first discrete element (A), and wherein
the second transfer unit (8) comprises at least a second working unit (9) movable along a second direction (R8) transverse to the second feed path (PB) for moving the second discrete element (B),
the first movable working unit (4) translating the first discrete element (A) along the first transverse direction (R3) and the second movable working unit (9) translating the second discrete element along the second transverse direction (R8).

14. The machine according to any one of claims 11 to 13, wherein the first transfer unit (3) rotates about a first axis of rotation (R3) transverse to the first feed path (PA) and the second transfer unit (8) rotates about a second axis of rotation (R8) transverse to the second feed path (PB).

15. The machine according to any one of claims 11 to 14, comprising a first acceleration unit (5) located downstream of the first transfer unit (3) and upstream of the first applicator station (7) along the first feed path (PA) for adjusting a transfer speed of the first discrete elements (A) before they are applied on the support web (W), and
a second acceleration unit (10) located downstream of the second transfer unit (8) and upstream of the second applicator station (12) along the second feed path (PB) for adjusting a transfer speed of the second discrete elements (B) before they are applied on the support web (W).

16. The machine according to any one of claims 11 to 15, wherein the adjustment device (14) comprises a first rotary drum (15) at the first applicator station (7), a second rotary drum (16) at the second applicator station (12), at least one adjustment drum (17) for adjusting the third feed path (PW) interposed between the first and the second rotary drum (15, 16), the support web (W) being at least partly wound round the first rotary drum (15), the second rotary drum (16) and the adjustment drum (17), the adjustment drum (17) being movable relative to the first rotary drum (15) and the second rotary drum (16) between a first operating position and a second operating position corresponding respectively to a maximum length of the third feed path (PW) between the first applicator station (7) and the second applicator station (12), and a minimum length of the third feed path (PW) between the first applicator station (7) and the second applicator station (12).

17. The machine according to claim 16, wherein the first rotary drum (15), the second rotary drum (16) and the adjustment drum (17) are suction drums for holding the support web (W) and the first and second discrete elements (A, B) on the support web (W).

18. The machine according to claim 17, comprising, between the first rotary drum (15) and the adjustment drum (17), a first suction box (20) at a first stretch (18) of the third feed path (PW) and comprising, between the adjustment drum (17) and the second rotary drum (16), a second suction box (21) at a second stretch (19) of the third feed path (PW), the whole of the third feed path (PW) between the first applicator station (7) and the second applicator station (12) being provided with suction for holding the first discrete element (A) and/or the second discrete element (B) on the support web (W).

19. The machine according to any one of claims 11 to 18, configured to implement the method according to any one of claims 1 to 10.

## Patentansprüche

1. Verfahren zur Herstellung absorbierender Hygieneartikel (100), die jeweils ein erstes Einzelelement (A) und ein zweites Einzelelement (B) umfassen, wobei das Verfahren Folgendes umfasst:
Zuführen einer Abfolge (SA) von ersten Einzelelementen (A) entlang eines ersten Zuführungswegs (PA);
Zuführen einer Abfolge (SB) von zweiten Einzelelementen (B) entlang eines zweiten Zuführungswegs (PB);
Auflegen eines jeden ersten Einzelelements (A) der Abfolge (SA) von ersten Einzelelementen (A) auf eine Stützbahn (W), aufweisend eine Längsachse und bewegbar in eine Zuführungsrichtung (VW) entlang eines dritten Zuführungswegs (PW), wobei die ersten Einzelelemente (A) auf die Stützbahn (W) an einer ersten Auflegestation (7) aufgelegt werden;
Auflegen eines jeden zweiten Einzelelements (B) der Abfolge (SB) von zweiten Einzelelementen (B) auf die Stützbahn (W) an einer zweiten Auflegestation (12), die in der Zuführungsrichtung (VW) der Stützbahn (W) stromabwärts der ersten Auflegestation (7) befindlich ist, wobei das Verfahren **dadurch gekennzeichnet ist, dass** es das Einstellen einer Länge des dritten Zuführungswegs (PW) zwischen der ersten Auflegestation (7) und der zweiten Auflegestation (12) umfasst, sodass jedes zweite Einzelelement (B) auf die Stützbahn (W) an einer vordefinierten Position relativ zu einem jeweiligen ersten Einzelelement (A) aufgelegt wird, wobei die Länge des dritten Zuführungswegs (PW) zwischen der ersten Auflegestation (7) und der zweiten Auflegestation (12) eine Länge der Stützbahn (W) zwischen der ersten Auflegestation (7) und der zweiten Auflegestation (12) bestimmt.

2. Verfahren nach Anspruch 1, wobei das erste und das zweite Einzelelement (A, B) auf die Stützbahn (W) derart aufgelegt werden, dass jedes zweite Einzelelement (B) symmetrisch zu einem entsprechenden ersten Einzelelement (A) um die Längsachse der Stützbahn (W) ist.

3. Verfahren nach Anspruch 1 oder 2, umfassend das Schneiden einer durchgehenden Bahn (AB) stromaufwärts des ersten und des zweiten Zuführungswegs (PA, PB), um die ersten Einzelelemente (A) und die zweiten Einzelelemente (B) aus der durchgehenden Bahn (AB) herzustellen, und Zuführen der ersten Einzelelemente (A) zum ersten Zuführungsweg (PA) und der zweiten Einzelelemente (B) zum zweiten Zuführungsweg (PB).

4. Verfahren nach Anspruch 3, wobei das Schneiden der durchgehenden Bahn (AB) eine Abfolge von Schnitten quer zu einer Hauptausdehnungsrichtung der durchgehenden Bahn (AB) erzeugt.

5. Verfahren nach einem der vorhergehenden Ansprüche, umfassend das Verschieben der ersten Einzelelemente (A) entlang des ersten Zuführungswegs (PA) in eine erste Richtung (R2) quer zum ersten Zuführungsweg (PA) und/oder das Verschieben der zweiten Einzelelemente (B) entlang des zweiten Zuführungswegs (PB) in eine zweite Richtung (R8) quer zum zweiten Zuführungsweg (PB).

6. Verfahren nach einem der vorhergehenden Ansprüche, umfassend das Beschleunigen der ersten Einzelelemente (A) entlang des ersten Zuführungswegs (PA) und/oder das Beschleunigen der zweiten Einzelelemente (B) entlang des zweiten Zuführungswegs (PB).

7. Verfahren nach einem der vorhergehenden Ansprüche, umfassend das Halten der ersten Einzelelemente (A) auf der Stützbahn (W) durch Ansaugen zumindest zwischen der ersten Auflegestation (7) und der zweiten Auflegestation (12) .

8. Verfahren nach einem der vorhergehenden Ansprüche, wobei das erste Einzelelement (A) eine erste Seitenplatte des absorbierenden Hygieneartikels (100) ist und das zweite Einzelelement (B) eine zweite Seitenplatte des absorbierenden Hygieneartikels (100) ist.

9. Verfahren nach einem der vorhergehenden Ansprüche, wobei die Summe einer Länge des ersten Einzelelements (A), gemessen entlang der Stützbahn (W), plus eine Länge des zweiten Einzelelements (B), gemessen entlang der Stützbahn (W), von der Länge des absorbierenden Hygieneartikels (100), gemessen entlang der Stützbahn (W), verschieden ist.

10. Verfahren nach einem der vorhergehenden Ansprüche, wobei das Einstellen der Länge des dritten Zuführungswegs (PW) zwischen der ersten Auflegestation (7) und der zweiten Auflegestation (12) abhängig von einer vordefinierten Auflageposition des zweiten Einzelelements (B) relativ zum ersten Einzelelement (A) entlang der Stützbahn (W) durchgeführt wird.

11. Maschine zur Herstellung absorbierender Hygieneartikel (100), umfassend:
eine erste Übergabeeinheit (3), die zumindest teilweise einen ersten Zuführungsweg (PA) für eine Abfolge (SA) erster Einzelelemente (A), die entlang des ersten Zuführungswegs (PA) bewegbar sind, definiert;
eine zweite Übergabeeinheit (8), die zumindest teilweise einen zweiten Zuführungsweg (PB) für eine Abfolge (SB) zweiter Einzelelemente (A), die entlang des zweiten Zuführungswegs (PA) bewegbar sind, definiert;
eine erste Auflegestation (7), die stromabwärts der ersten Übergabeeinheit (3) entlang des ersten Zuführungswegs (PA) befindlich ist, um jedes erste Einzelelement (A) der Abfolge (SA) erster Einzelelemente (A) auf eine Stützbahn (W), aufweisend eine Längsachse und bewegbar in eine Zuführungsrichtung (VW) entlang eines dritten Zuführungswegs (PW), aufzulegen;
eine zweite Auflegestation (12), die stromabwärts der ersten Auflegestation (7) in der Zuführungsrichtung (VW) der Stützbahn (W) und stromabwärts der zweiten Übergabeeinheit (8) entlang des zweiten Übergabewegs (PB) befindlich ist, um jedes zweite Einzelelement (B) der Abfolge (SB) zweiter Einzelelemente (B) auf die Stützbahn (W) aufzulegen, wobei die Maschine **dadurch gekennzeichnet ist, dass** sie eine Einstellvorrichtung (14) zum Einstellen einer Länge des dritten Zuführungswegs (PW) zwischen der ersten Auflegestation (7) und der zweiten Auflegestation (12) umfasst, sodass jedes zweite Einzelelement (B) auf die Stützbahn (W) an einer vordefinierten Position relativ zu einem jeweiligen ersten Einzelelement (A) aufgelegt wird, wobei die Einstellvorrichtung (14) zumindest teilweise den dritten Zuführungsweg (PW) definiert, wobei die Länge des dritten Zuführungswegs (PW) zwischen der ersten Auflegestation (7) und der zweiten Auflegestation (12) eine Länge der Stützbahn (W) zwischen der ersten Auflegestation (7) und der zweiten Auflegestation (12) definiert.

12. Maschine nach Anspruch 11, umfassend eine Schneideinheit (2), die stromaufwärts der ersten und der zweiten Übergabeeinheit (3, 8) befindlich ist, um eine durchgehende Bahn (AB) stromaufwärts des ersten und des zweiten Zuführungswegs (PA, PB) zu schneiden, um die ersten Einzelelemente (A) und die zweiten Einzelelemente (B) aus der durchgehenden Bahn (AB) herzustellen, wobei die Schneideinheit (2) und die erste und die zweite Übergabeeinheit (3, 8) relativ zueinander derart positioniert sind, dass die Schneideinheit (2) die ersten Einzelelemente (A) dem ersten Zuführungsweg (PA) und die zweiten Einzelelemente (B) dem zweiten Zuführungsweg (PB) zuführt.

13. Maschine nach Anspruch 11 oder 12, wobei die erste Übergabeeinheit (3) mindestens eine erste Arbeitseinheit (4) umfasst, die entlang einer ersten Richtung (R3) quer zum ersten Zuführungsweg (PA) bewegbar ist, um das erste Einzelelement (A) zu bewegen, und wobei
die zweite Übergabeeinheit (8) mindestens eine zweite Arbeitseinheit (9) umfasst, die entlang einer zweiten Richtung (R8) quer zum zweiten Zuführungsweg (PB) bewegbar ist, um das zweite Einzelelement (B) zu bewegen, wobei die erste bewegbare Arbeitseinheit (4) das erste Einzelelement (A) entlang der ersten Querrichtung (R3) verschiebt und die zweite bewegbare Arbeitseinheit (9) das zweite Einzelelement entlang der zweiten Querrichtung (R8) verschiebt.

14. Maschine nach einem der Ansprüche 11 bis 13, wobei sich die erste Übergabeeinheit (3) um eine erste Rotationsachse (R3) quer zum ersten Zuführungsweg (PA) dreht und sich die zweite Übergabeeinheit (8) quer zum zweiten Zuführungsweg (PB) um eine zweite Rotationsachse (R8) dreht.

15. Maschine nach einem der Ansprüche 11 bis 14, umfassend eine erste Beschleunigungseinheit (5), die stromabwärts der ersten Übergabeeinheit (3) und stromaufwärts der ersten Auflegestation (7) entlang des ersten Zuführungswegs (PA) befindlich ist, um eine Übergabegeschwindigkeit der ersten Einzelelemente (A) einzustellen, bevor diese auf die Stützbahn (W) aufgelegt werden, und
eine zweite Beschleunigungseinheit (10), die stromabwärts der zweiten Übergabeeinheit (8) und stromaufwärts der zweiten Auflegestation (12) entlang des zweiten Zuführungswegs (PB) befindlich ist, um eine Übergabegeschwindigkeit der zweiten Einzelelemente (B) einzustellen, bevor diese auf die Stützbahn (W) aufgelegt werden.

16. Maschine nach einem der Ansprüche 11 bis 15, wobei die Einstellvorrichtung (14) eine erste Drehtrommel (15) an der ersten Auflegestation (7), eine zweite Drehtrommel (16) an der zweiten Auflegestation (12), mindestens eine Einstelltrommel (17) zum Einstellen des dritten Zuführungswegs (PW), die zwischen der ersten und der zweiten Drehtrommel (15, 16) eingesetzt ist, umfasst, wobei die Stützbahn (W) zumindest teilweise rund um die erste Drehtrommel (15), die zweite Drehtrommel (16) und die Einstelltrommel (17) gewunden ist, wobei die Einstelltrommel (17) relativ zur ersten Drehtrommel (15) und der zweiten Drehtrommel (16) zwischen einer ersten Betriebsposition und einer zweiten Betriebsposition entsprechend jeweils einer Höchstlänge des dritten Zuführungswegs (PW) zwischen der ersten Auflegestation (7) und der zweiten Auflegestation (12) und einer Mindestlänge des dritten Zuführungswegs (PW) zwischen der ersten Auflegestation (7) und der zweiten Auflegestation (12) bewegbar ist.

17. Maschine nach Anspruch 16, wobei die erste Drehtrommel (15), die zweite Drehtrommel (16) und die Einstelltrommel (17) Saugtrommeln sind, um die Stützbahn (W) und die ersten und die zweiten Einzelelemente (A, B) auf der Stützbahn (W) zu halten.

18. Maschine nach Anspruch 17, umfassend zwischen der ersten Drehtrommel (15) und der Einstelltrommel (17) einen ersten Saugkasten (20) an einem ersten Teilstück (18) des dritten Zuführungswegs (PW) und umfassend zwischen der Einstelltrommel (17) und der zweiten Drehtrommel (16) einen zweiten Saugkasten (21) an einem zweiten Teilstück (19) des dritten Zuführungswegs (PW), wobei die Gesamtheit des dritten Zuführungswegs (PW) zwischen der ersten Auflegestation (7) und der zweiten Auflegestation (12) mit einer Ansaugung versehen ist, um das erste Einzelelement (A) und/oder das zweite Einzelelement (B) auf der Stützbahn (W) zu halten.

19. Maschine nach einem der Ansprüche 11 bis 18, die ausgelegt ist, um das Verfahren nach einem der Ansprüche 1 bis 10 umzusetzen.

## Revendications

1. Procédé de fabrication d'articles sanitaires absorbants (100), comprenant chacun un premier élément distinct (A) et un second élément distinct (B), le procédé comprenant :
alimenter une succession (SA) de premiers éléments distincts (A) le long d'un premier trajet d'alimentation (PA) ;
alimenter une succession (SB) de seconds éléments distincts (B) le long d'un deuxième trajet d'alimentation (PB) ;
appliquer chaque premier élément distinct (A) de la succession (SA) des premiers éléments distincts (A) sur une bande de support (W) ayant un axe longitudinal et mobile dans une direction d'alimentation (VW) le long d'un troisième trajet d'alimentation (PW), les premiers éléments distincts (A) étant appliqués sur la bande de support (W) au niveau d'une première station d'application (7) ;
appliquer chaque second élément distinct (B) de la succession (SB) de seconds éléments distincts (B) sur la bande de support (W) au niveau d'une seconde station d'application (12) située en aval de la première station d'application (7) dans la direction d'alimentation (VW) de la bande de support (W), le procédé étant **caractérisé en ce qu'**il comprend régler une longueur du troisième trajet d'alimentation (PW) entre la première station d'application (7) et la seconde station d'application (12), de sorte que chaque second élément distinct (B) est appliqué sur la bande de support (W) dans une position prédéfinie par rapport à un premier élément distinct respectif (A), la longueur du troisième trajet d'alimentation (PW) entre la première station d'application (7) et la seconde station d'application (12) déterminant une longueur de la bande de support (W) entre la première station d'application (7) et la seconde station d'application (12).

2. Procédé selon la revendication 1, dans lequel le premier et le second élément distinct (A, B) sont appliqués sur la bande de support (W) de telle manière que chaque second élément distinct (B) est symétrique par rapport à un premier élément distinct correspondant (A) autour de l'axe longitudinal de la bande de support (W).

3. Procédé selon la revendication 1 ou 2, comprenant couper une bande continue (AB) en amont du premier et du deuxième trajet d'alimentation (PA, PB) pour réaliser les premiers éléments distincts (A) et les seconds éléments distincts (B) à partir de la bande continue (AB) et alimenter les premiers éléments distincts (A) vers le premier trajet d'alimentation (PA) et les seconds éléments distincts (B) vers le deuxième trajet d'alimentation (PB).

4. Procédé selon la revendication 3, dans lequel couper la bande continue (AB) produit une succession de coupes transversales à une direction principale d'extension de la bande continue (AB).

5. Procédé selon l'une quelconque des revendications précédentes, comprenant translater les premiers éléments distincts (A) le long du premier trajet d'alimentation (PA) dans une première direction (R2) transversale au premier trajet d'alimentation (PA) et/ou translater les seconds éléments distincts (B) le long du deuxième trajet d'alimentation (PB) dans une seconde direction (R8) transversale au deuxième trajet d'alimentation (PB).

6. Procédé selon l'une quelconque des revendications précédentes, comprenant accélérer les premiers éléments distincts (A) le long du premier trajet d'alimentation (PA) et/ou accélérer les seconds éléments distincts (B) le long du deuxième trajet d'alimentation (PB).

7. Procédé selon l'une quelconque des revendications précédentes, comprenant maintenir les premiers éléments distincts (A) sur la bande de support (W) par aspiration au moins entre la première station d'application (7) et la seconde station d'application (12).

8. Procédé selon l'une quelconque des revendications précédentes, dans lequel le premier élément distinct (A) est un premier panneau latéral de l'article sanitaire absorbant (100) et le second élément distinct (B) est un second panneau latéral de l'article sanitaire absorbant (100) .

9. Procédé selon l'une quelconque des revendications précédentes, dans lequel la somme d'une longueur du premier élément distinct (A), mesurée le long de la bande de support (W), plus une longueur du second élément distinct (B), mesurée le long de la bande de support (W), est différente de la longueur de l'article sanitaire absorbant (100), mesurée le long de la bande de support (W).

10. Procédé selon l'une quelconque des revendications précédentes, dans lequel régler la longueur du troisième trajet d'alimentation (PW) entre la première station d'application (7) et la seconde station d'application (12) est effectué en fonction d'une position d'application prédéfinie du second élément distinct (B) par rapport au premier élément distinct (A) le long de la bande de support (W).

11. Machine de fabrication d'articles sanitaires absorbants (100), comprenant :
une première unité de transfert (3) définissant au moins partiellement un premier trajet d'alimentation (PA) pour une succession (SA) de premiers éléments distincts (A) mobiles le long du premier trajet d'alimentation (PA) ;
une seconde unité de transfert (8) définissant au moins partiellement un deuxième trajet d'alimentation (PB) pour une succession (SB) de seconds éléments distincts (B) mobiles le long du deuxième trajet d'alimentation (PB) ;
une première station d'application (7) située en aval de la première unité de transfert (3) le long du premier trajet d'alimentation (PA) pour appliquer chaque premier élément distinct (A) de la succession (SA) de premiers éléments distincts (A) sur une bande de support (W) ayant un axe longitudinal et mobile dans une direction d'alimentation (VW) le long d'un troisième trajet d'alimentation (PW) ;
une seconde station d'application (12) située en aval de la première station d'application (7) dans la direction d'alimentation (VW) de la bande de support (W) et en aval de la seconde unité de transfert (8) le long du deuxième trajet d'alimentation (PB) pour appliquer chaque second élément distinct (B) de la succession (SB) de seconds éléments distincts (B) sur la bande de support (W), la machine étant **caractérisée en ce qu'**elle comprend un dispositif de réglage (14) pour régler une longueur du troisième trajet d'alimentation (PW) entre la première station d'application (7) et la seconde station d'application (12), de sorte que chaque second élément distinct (B) est appliqué sur la bande de support (W) dans une position prédéfinie par rapport à un premier élément distinct respectif (A), le dispositif de réglage (14) définissant au moins partiellement le troisième trajet d'alimentation (PW), la longueur du troisième trajet d'alimentation (PW) entre la première station d'application (7) et la seconde station d'application (12) déterminant une longueur de la bande de support (W) entre la première station d'application (7) et la seconde station d'application (12).

12. Machine selon la revendication 11, comprenant une unité de coupe (2) située en amont de la première et de la seconde unité de transfert (3, 8) pour couper une bande continue (AB) en amont du premier et du deuxième trajet d'alimentation (PA, PB) pour réaliser les premiers éléments distincts (A) et les seconds éléments distincts (B) à partir de la bande continue (AB), l'unité de coupe (2) et les première et seconde unités de transfert (3, 8) étant positionnées l'une par rapport à l'autre de telle manière que l'unité de coupe (2) alimente les premiers éléments distincts (A) vers le premier trajet d'alimentation (PA) et les seconds éléments distincts (B) vers le deuxième trajet d'alimentation (PB).

13. Machine selon la revendication 11 ou 12, dans laquelle la première unité de transfert (3) comprend au moins une première unité de travail (4) mobile le long d'une première direction (R3) transversale au premier trajet d'alimentation (PA) pour déplacer le premier élément distinct (A), et dans laquelle
la seconde unité de transfert (8) comprend au moins une seconde unité de travail (9) mobile le long d'une seconde direction (R8) transversale au deuxième trajet d'alimentation (PB) pour déplacer le second élément distinct (B),
la première unité de travail mobile (4) translatant le premier élément distinct (A) le long de la première direction transversale (R3) et la seconde unité de travail mobile (9) translatant le second élément distinct le long de la seconde direction transversale (R8).

14. Machine selon l'une quelconque des revendications 11 à 13, dans laquelle la première unité de transfert (3) tourne autour d'un premier axe de rotation (R3) transversal au premier trajet d'alimentation (PA) et la seconde unité de transfert (8) tourne autour d'un second axe de rotation (R8) transversal au deuxième trajet d'alimentation (PB).

15. Machine selon l'une quelconque des revendications 11 à 14, comprenant une première unité d'accélération (5) située en aval de la première unité de transfert (3) et en amont de la première station d'application (7) le long du premier trajet d'alimentation (PA) pour régler une vitesse de transfert des premiers éléments distincts (A) avant qu'ils ne soient appliqués sur la bande de support (W), et
une seconde unité d'accélération (10) située en aval de la seconde unité de transfert (8) et en amont de la seconde station d'application (12) le long du deuxième trajet d'alimentation (PB) pour régler une vitesse de transfert des seconds éléments distincts (B) avant qu'ils ne soient appliqués sur la bande de support (W).

16. Machine selon l'une quelconque des revendications 11 à 15, dans laquelle le dispositif de réglage (14) comprend un premier tambour rotatif (15) au niveau de la première station d'application (7), un second tambour rotatif (16) au niveau de la seconde station d'application (12), au moins un tambour de réglage (17) pour régler le troisième trajet d'alimentation (PW) interposé entre le premier et le second tambour rotatif (15, 16), la bande de support (W) étant au moins partiellement enroulée autour du premier tambour rotatif (15), du second tambour rotatif (16) et du tambour de réglage (17), le tambour de réglage (17) étant mobile par rapport au premier tambour rotatif (15) et au second tambour rotatif (16) entre une première position de fonctionnement et une seconde position de fonctionnement correspondant respectivement à une longueur maximale du troisième trajet d'alimentation (PW) entre la première station d'application (7) et la seconde station d'application (12), et une longueur minimale du troisième trajet d'alimentation (PW) entre la première station d'application (7) et la seconde station d'application (12).

17. Machine selon la revendication 16, dans laquelle le premier tambour rotatif (15), le second tambour rotatif (16) et le tambour de réglage (17) sont des tambours d'aspiration pour maintenir la bande de support (W) et les premier et second éléments distincts (A, B) sur la bande de support (W).

18. Machine selon la revendication 17, comprenant, entre le premier tambour rotatif (15) et le tambour de réglage (17), une première boîte d'aspiration (20) au niveau d'un premier tronçon (18) du troisième trajet d'alimentation (PW) et comprenant, entre le tambour de réglage (17) et le second tambour rotatif (16), une seconde boîte d'aspiration (21) au niveau d'un second tronçon (19) du troisième trajet d'alimentation (PW), l'ensemble du troisième trajet d'alimentation (PW) entre la première station d'application (7) et la seconde station d'application (12) étant pourvue d'une aspiration pour maintenir le premier élément distinct (A) et/ou le second élément distinct (B) sur la bande de support (W).

19. Machine selon l'une quelconque des revendications 11 à 18, configurée pour mettre en œuvre le procédé selon l'une quelconque des revendications 1 à 10.
